Europäisches Patentamt

European Patent Office

Office européen des brevets

(19)

(11) Publication number: **0 178 764**
A1

(12) **EUROPEAN PATENT APPLICATION**

(21) Application number: **85306061.4**

(22) Date of filing: **27.08.85**

(51) Int. Cl.⁴: **C 12 N 15/00**
**C 12 P 21/00, C 12 N 1/20**
**//C12R1:19, C12R1:22**

(30) Priority: **28.08.84 US 644998**

(43) Date of publication of application:
**23.04.86 Bulletin 86/17**

(84) Designated Contracting States:
**AT BE CH DE FR GB IT LI LU NL SE**

(71) Applicant: **GENEX CORPORATION**
**6110 Executive Boulevard**
**Rockville Maryland 20852(US)**

(72) Inventor: **Anderson, David Martin**
**13501, Grenobile Drive**
**Rockville Maryland 20853(US)**

(74) Representative: **Holmes, Michael John et al,**
**Frank B. Dehn & Co. Imperial House 15-19 Kingsway**
**London WC2B 6UZ(GB)**

(54) **Method of stabilizing a host microorganism/expression vector system for large scale production of proteins.**

(57) This invention provides a method of stabilizing a host microorganism / expression vector system for large scale production of a protein which comprises transforming an auxotrophic strain of said microorganism, having at least one mutation in the tryptophan operon such that the cells are unable to produce sufficient tryptophan to survive or grow, with a recombinant vector which comprises (a) a gene which encodes a protein of interest and is under the control of regulatory sequences for expression and (b) one or more functional *trp* genes, under the control of regulatory sequences for expression, which at least complement the mutation(s) in the tryptophan operon of the host cells thereby enabling the said host cells to synthesize tryptophan.

EP 0 178 764 A1

Croydon Printing Company Ltd

# METHOD OF STABILIZING A HOST MICROORGANISM/
EXPRESSION VECTOR SYSTEM FOR LARGE SCALE
PRODUCTION OF PROTEINS

This invention relates to a method of stabilizing a host microorganism / expression vector system for large scale production of proteins by offsetting the selective pressure against cells which contain production plasmids.

Advances in molecular biology techniques have made it possible to introduce genetic information from any source in the form of DNA into vectors that replicate as phage or plasmids in prokaryotic organisms. The genetic information can be an in vitro generated DNA copy of mRNA, as is frequently prepared from eukaryotic organisms, DNA isolated directly from the genome, as is the preferred method for prokaryotic organisms, or chemically synthesized DNA. In applied research applications of this methodology, the goal generally is to overproduce a protein that is encoded by the inserted genetic information or a chemical whose synthesis depends in part on enzymatic activity of a protein encoded by the inserted genetic information. This protein production is accomplished by large scale fermentation of the genetically engineered microorganisms.

The actual high level protein production, or expression of the gene, is not a normal or preferred method by which the cell utilizes its available energies. As a result, there is a strong selective pressure for transformed organisms to revert, i.e. to expel all or part of the DNA inserted by genetic engineering techniques, and fermentations which contain revertent organisms, when grown, can show a constantly diminishing percentage of transformed organisms. Therefore, a strong selective pressure must be applied for the maintenance of the transformed state. In experimental work which involves small-scale petri dish and flask cultures, vector maintenance selection generally is by the use of vectors which, in addition to the gene for the product to be produced, contain a gene conferring resistance to an antibiotic to which the host organism is normally susceptible. The subsequent addition of the antibiotic to the growth medium favors the propagation of cells that possess the gene-containing plasmid or phage over those not possessing resistance to the antibiotic. This selective pressure effectively controls the growth of revertent vector free strains and allows the production of nearly pure, transformed cultures. While this is an adequate and effective method for selecting and cloning transformed organisms on a laboratory scale, the price of most effective antibiotics makes their use in industrial-scale fermentors expensive. This expense is often increased since fermentors produce microorganisms in greater cell density than is the case with laboratory flask cultures, requiring higher concentrations of antibiotics. Cells overproducing a protein or chemical of interest will generally grow much slower than non-transformed cells. Thus, unless antibiotic concentration is maintained at a high level, cells that have lost the vector (and therefore, the ability to produce the protein of interest) can rapidly grow to become the predominant species lowering overall productivity of the desired protein.

A partial solution to the problem has been to regulate the expression of the inserted genes such that protein production is initiated only after the cell mass is largely developed in a fermentation, either through inducible transcription systems or vector copy number.

In that way the great selective growth advantage for cells that have lost the vector is much decreased until induction. However, maintenance of the vector even without gene expression can be disadvantagous to cells relative to non-vector containing cells. For example, E. coli cells containing pBR322 based plasmids without antibiotic or other selection will generate revertants at a rate of approximately 1% per generation even without high level gene expression. Also, after induction of protein synthesis there is the large growth advantage for cells that have lost the vector and some potential yield loss.

Another method that is helpful for vector stability is to increase the inherent stability or partition proficiency of the vector (e.g. see European Patent Application 83303195.8) by addition of DNA segments that contain a partition locus or by originally using vectors that already contain a partition locus. Such DNA segments are involved in the partition or transfer of plasmid to both daughter cells after a division. Use of this method will decrease the rate of revertant formation, but it does not eliminate the problem and revertants once formed still have a great selective advantage for growth.

An existing plasmid stabilization method suitable for industrial use involves placing genes for streptomycin independence on a plasmid in order to stabilize that plasmid in a streptomycin dependent host (Miwa et al. U.S. Patent No. 4,371,615). However, cell mutants that are dependent on streptomycin are capable of reverting to streptomycin independence or sensitivity. Also, since streptomycin binds to a ribosomal protein, some of the ribosomes necessary for protein synthesis in the complemented host would be defective thus decreasing the efficiency of protein synthesis. There remains, therefore, a need for an alternative method of exerting selective pressure in favour of the growth of transformed organisms that is effective in large scale fermentations and inexpensive to implement.

## SUMMARY OF THE INVENTION

Acccording to one aspect of the present invention, there is provided a method for the large scale production of a protein by a microorganism which comprises transforming an auxotrophic strain of said microorganism, having at least one mutation in the tryptophan operon such that the cells are unable to produce sufficient tryptophan to survive or grow, with a recombinant vector which comprises (a) a gene which encodes a protein of interest and is under the control of regulatory sequences for expression and (b) one or more functional trp genes, under the control of regulatory sequences for expression, which at least complement the mutation(s) in the tryptophan operon of the host cells thereby enabling the said host cells to synthesize tryptophan, and growing the transformed cells under gene expressing conditions in nutrient medium therefor substantially free of tryptophan.

For example, in a rich medium wherein tryptophan has been degraded, trp⁻ microbial cells with, for example, a mutation in one or more genes of the trp operon can be enabled to grow by complementing the mutated trp gene(s) with the appropriate unmutated trp gene(s) carried on a recombinant vector suitable for expression of a desired foreign protein. This places selective pressure on the transformed cells to retain the vector, for the protein product(s) of the functional vector-borne trp gene(s) is/are essential for the synthesis of tryptophan and thus to enable the host cells to survive and grow. This, in turn, allows expression of the desired foreign protein also encoded by the vector.

## BRIEF DESCRIPTION OF FIGURES

Figure 1 is a schematic representation of the construction of plasmid pGX2257, a prochymosin expression vector with trpED genes to complement a host cell trpED102 mutation.

Figure 2 is a schematic representation of the construction of plasmid pGX2237, a serine hydroxymethyltransferase expression vector carrying the trp operon genes to complement trp mutations in host cells.

## DETAILED DESCRIPTION OF THE INVENTION

The method of the present invention is applicable to the stabilization and selection of prokaryotic cell lines which are transformed to produce enzymes, hormones and other proteins of interest, and which are therefore at a competitive disadvantage relative to untransformed host cells.

In accordance with this invention, a host cell line is selected with, or mutated to provide, a mutation in the tryptophan operon, e.g. in one or more genes of the cells which code for tryptophan synthesis, so that tryptophan is a necessary component in the growth medium for growth or survival of the cells. The defect created by the mutation may either be one which causes improper translation and, therefore, a defective protein product, or a defect which interrupts either transcription or translation; either type of defect will result in the cells' failure to produce tryprophan. In as much as tryptophan is necessary for the cells' maintenance or growth, the survival of the cells is dependent upon obtaining that component from another source.

Genetic mutations may be introduced in the genome of a microorganism in a number of ways, depending upon the nature of the organism. Mutation techniques are well known and include, for example, exposing a microorganism to a chemical mutagen, such as nitrous acid, N-methyl-N'-nitro-N-nitrosoguanidine, ethylmethane sulphonate, 5-bromouracil, hydroxylamine, nitrogen or a sulphur mustard. Exposing the cells to radiation such as ultraviolet light, X-rays or gamma radiation can also be employed. Such techniques are well known, and are described for example, in "Basic Bacteriology," La Manna and Mallette, Second Edition, the Williams & Williams Company, Baltimore, MD, 1959, on pages 646 to 649.

Insertional inactivation of genes or regulatory sequences in the trp operon may also be used to obtain the auxotrophic host cells used in this invention. Foreign DNA may be introduced into the trp operon, for example, by way of various viruses, transducing phages and transposons. Viruses which are capable of incorporating exogenous nucleotide sequences into cellular DNA (e.g., the bacteriophages mu and Pl), are generally applicable. A general discussion of transduction by such viruses is found in Pelczar, M. and R. Reid, Microbiology, 3rd Ed., McGraw Hill, Inc. (1972) pp. 228-229. Translocatable elements (transposons), stretches of DNA which can move as discrete genetic and physical entities from one position in a prokaryotic genome to another position in that or a different genome, also may alter readthrough (See Kleckner, Cell 11, 11 (1977)). Organisms modified by any of the above-described mutational processes then may be screened for their ability to produce the essential component, and those which are unable to produce, i.e., those with a host lethal mutation, then can be grown only in the presence of the essential missing nutrient.

In a method according to this invention, cells of a host microorganism may, for example, be mutated such that one or more genes of the tryptophan (trp) operon are deleted. Cells carrying a desired mutation in the trp operon are selected. Then an unmutated trp gene or genes corresponding to the mutated trp gene(s) is/are inserted into a vector which also carries a gene which encodes for production of a desired foreign protein, such as, for example, prochymosin or serine hydroxymethyl transferase (SHMT). Both the trp gene(s) and the gene encoding the foreign protein are carried on the vector under the control of sequences necessary for expression, including a promoter/operator, a ribosome binding site, and a translation initiation codon. The vector may be either a plasmid or a phage.

The vector then transforms the trp deficient host cells, and the transformed cells are cultured in a medium substantially lacking in tryptophan. The cells transformed with the vector carrying the trp gene(s) complementary to the mutated trp gene(s) thus are placed at a competitive advantage in comparison to untransformed cells. The transformed cells flourish, while the untransformed cells cannot grow at all, and large quantities of the desired protein can be obtained.

In carrying out the method of this invention, it is desirable that the host lethal genetic defect be one for which reversion cannot or is unlikely to occur. It also is desirable that the gene which codes for the production of the desired material be carried on the vector in such a way that expression can be induced at a desired time. In order to prevent the possibility of recombination of the homologous genes and deletion of plasmid DNA, it also is desirable that the gene(s) used to stabilize the vector-host cell system be present in only one location on the vector.

The method of stabilizing a host microorganism/ expression vector system of this invention has a number of advantages in comparison to previous efforts to increase the selective pressure on cells which contain expression vectors in order to obtain large quantities of a desired foreign protein. For example, by the procedure

0178764

of this invention, the cells are grown in media substantially without tryptophan. Tryptophan is relatively unstable; thus, it can be selectively destroyed in a rich medium containing many amino acids without harming most of the other components of the medium. Thus, this method does not preclude the use of a complex, rich medium as would a complementation stabilization scheme involving the majority of other amino acid operons. For instance, tryptophan can easily be destroyed in protein hydrolysates by acid hydrolysis. Tryptophan also can be selectively destroyed in rich media by the addition of the enzyme tryptophanase.

In addition, tryptophan manufacture by cells requires more energy than the production of any other amino acid. The biosynthesis of tryptophan thus is tightly controlled (Platt, T. (1978) in Miller, J.H. and W.F. Reznitzoff, eds., The Operon, pp. 263-302, Cold Spring Harbor Laboratory, N.Y.) Provided that normal regulatory mechanisms are present, increasing the gene copy number of the tryptophan operon in a cell does not result in excess tryptophan synthesis and excretion out of the cell. Thus, the method of this invention would not result in undesirable cross-feeding of cells that have lost the recombinant vector.

Finally, this method is available for stabilizing a wide variety of host cell-recombinant vector systems such that large quantities of the desired protein can be expressed, for host cells defective in one or more genes of the tryptophan operon can be isolated easily in the production strain by either mutation and selection or transduction.

The following examples are intended to illustrate the present invention, but are not to be construed as limiting.

EXAMPLE I                               0178764

## Construction of a Compensating Plasmid Expressing a Foreign Protein (pGX2257 (trpED, trpB Δ2 prochymosin))

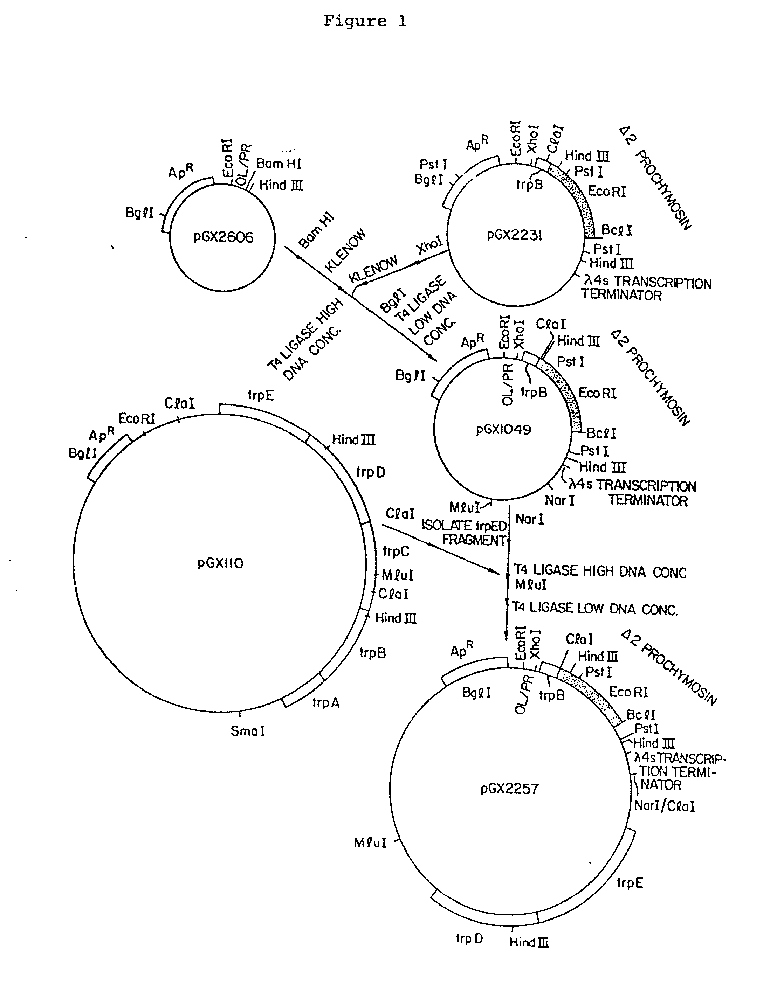

The process for the production of pGX2257 is illustrated in Figure 1. An E. coli host strain, GX1670, was transformed with plasmid pGX2231 and deposited with the U.S. Department of Agriculture, Agricultural Research Service in Peoria, Illinois, under access number NRRL B-15571 on 25 August 1983. This plasmid contains a nearly complete prochymosin gene (lacking the codons for the first two amino acids) fused in frame downstream to a 5' fragment of the E. coli trpB gene under the control of a trp/lac promoter. A restriction site susceptible to XhoI exists between the trp/lac promoter and the trpB fragment. Plasmid pGX2606, which was deposited in E. coli K12 GX1170 (λ) with the U.S. Department of Agriculture, Agriculture Research Service in Peoria, Illinois on 16 August 1983 under access number NRRL B-15551, contains a $\lambda O_L/P_R$ hybrid promoter/operator adjacent to a BamHI susceptible site and an ampicillin resistance gene.

Plasmid pGX2231 was digested with XhoI and the sticky ends filled with the Klenow fragment of DNA polymerase (Klenow, H., et al., Eur. J. Biochem., 22, 371 (1977)) in a reaction mixture containing dATP, dTTP, dGTP, and dCTP. Plasmid pGX2606 was digested with BamHI and the sticky ends again were filled with the Klenow fragment. The two DNAs were purified by phenol-chloroform extraction and ethanol precipitation, and ligated at high DNA concentration (approximately 200 µg/ml) using T₄ DNA ligase. This intermediate was then digested with BglI and the resultant mixture was extracted with phenol-chloroform and ethanol

Precipitated. A second ligation was performed, using $T_4$ ligase at low DNA concentration to form a plasmid pGX1049, which was used to transform E. coli strain GX1201 containing a defective λ lysogen with a cI857 mutation encoding a temperature sensitive repressor protein by selecting for cells exhibiting ampicillin resistance. Resistant cells were further screened for their ability to produce trpB-Δ2prochymosin after a temperature shift to 42°C. Production at elevated temperature indicates that the trpB-Δ2prochymosin gene is under the control of the $O_L/P_R$ promoter-operator in response to repressor protein coded by the cI857 gene.

Strain GX1201 (pGX1049) was grown at 30°C in shaker flasks containing LB medium containing ampicillin (100 μg/ml), and plasmid pGX1049 was isolated according to standard techniques and digested with NarI.

Plasmid pGX110, a plasmid that is virtually identical to pEP12 (Enger-Valk, B.E. et al. Gene 9: 69-135, 1980)contains a complete trp operon and two ClaI susceptible sites, including one in the trpC gene. The plasmid was digested with Cla I and the 4.86 kb DNA fragment containing the trpED genes and part of the trpC gene was isolated by electrophoresis on 0.85% agarose gel, electroelution, chromatography on DEAE cellulose (eluting solution was 1.0 M NaCl in $10^{-2}$ M Tris, $10^{-3}$ M EDTA pH 8.0) followed by ethanol precipitation.

The linearized pGX1049 and trpEDC DNA fragments were ligated using $T_4$ DNA ligase at high DNA concentration (no other prior treatment was necessary as NarI and ClaI generate the same sticky ends). Reformation into a circularized plasmid was accomplished by first digesting with MluI, which cleaves within trpC and in a non-essential location in the DNA derived from pGX1049, purification by phenol-chloroform extraction and ethanol precipitation, and ligation again with $T_4$ ligase

at low DNA concentrations (1-10 µg/ml). The ligation mixture was transformed into an E. coli strain containing the trpED102 mutation (Jackson, E. N. and Yanofsky, C., J. Molecular Bio., 71, 149 (1972)). Construction of this strain, E. coli GX1731, is described in Example 2. Transformants were identified by ampicillin resistance and the ability to grow in the absence of exogenous tryptophan. Prochymosin production was demonstrated following temperature shift induction, and the plasmid, isolated using standard techniques, was verified by restriction endonuclease digestion. The plasmid was designated pGX2257. The E. coli strain transformed by this plasmid, GX1731(pGX2257) was deposited with the U.S. Department of Agriculture, Agricultural Research Service in Peoria, Illinois, as NRRL No. B-15771 on 27 April 1984.

EXAMPLE II

Construction of Host Strain GX1731
Using P1 Transduction

The phage P1 transduction method basically described by Miller (Miller, J.H. Experiments in Molecular Genetics, Cold Spring Harbor Laboratory, 1972) was used. E. coli SP338 (F⁻, trpED102, thr, tna2), (Roeder and Somerville, Molecular and General Genetics, 176: 361-368 (1979) is available from the U.S. Department of Agriculture Northern Regional Research Center with accession number NRRL B-4569. A culture of SP338 was grown in LB medium containing 0.1M $MgSO_4$ and 0.05M $CaCl_2$ to an $A_{540}$ of 0.3. Then, 0.1ml aliquots of 1 to 10 and 1 to 100 dilutions of Pl$_{KC}$ lysate prepared by infection of E. coli GX1213 (F⁻,[λcI857 ΔBamΔH1], nadA::Tn10, his, ilv, Δ[chlD-pgl]) were added to 0.1 ml of SP338 cells and incubated at 37°C for 30 minutes. The cells were then pelleted by centrifugation, resuspended in approximately

0.1ml of LB, and spread onto LB agar plates containing 25µg/ml tetracycline. The plates were incubated at 30°C. Tetracycline resistant transductants were picked and grown in LB agar plus 25µg/ml tetracycline at 30°C. Cultures were streaked on TBM agar plates and spotted with lysates of λ and λvir phages (M. Ptashne in A.D. Hershey [ed.] The Bacteriophage Lambda, pp. 221-245, Cold Spring Harbor Laboratory, 1971). Duplicate sets of plates were prepared, one set incubated at 30°C and the other at 42°C. One isolate was selected which was resistant to λ at 30°C, but not resistant at 42°C and yet was lysed by λvir at both temperatures. Resistance to λ at 30°C but lysis at 42°C indicates the presence of the temperature sensitive cI857 lambda repressor in the chromosome. Lysis by λ vir (that is immune to repressor) is an additional control to verify that λ resistance has not been conferred by a mechanism unrelated to the cI gene product. The tryptophan and threonine mutations were confirmed via analysis of growth response on MinA 1% glucose agar plates with and without threonine and tryptophan at 20µg/ml at 30°C. The resulting strain was designated GX1729 (F⁻, trpED102, thr, tna2, [λcI857 ΔBamΔHI], Δ[chlD-pgl], nadA::Tn10).

| TBM | 1% tryptone | Minimal A agar 1.5% | |
|---|---|---|---|
| | .5% NaCl | $K_2HPO_4$ | 10.4g/l |
| | 1.5% agar | $KH_2PO_4$ | 4.5g/l |
| | .2% maltose | $(NH_4)_2SO_4$ | 1.0g/l |
| | 10mM $MgSO_4$ | sodium citrate · $2H_2O$ | 0.5g/l |
| | | $MgSO_4$ | 0.2g/l |

GX1729 was then transduced as described above using a Pl$_{KC}$ lysate prepared by infecting E. coli, American Type Culture Collection No. 27325 (wild type). The pelleted cells were washed once with 0.9% NaCl, resuspended in a minimal volume of 0.9% NaCl, and spread on MinA 1% glucose plates containing tryptophan at 20

μg/ml but no threonine. The plates were incubated at 30°C. Resulting isolates were checked to make sure they retained the same λ infection phenotype and tryptophan auxotrophy as GX1729 but no longer required threonine. The resulting isolate was designated GX1731 (F⁻, trpED102, tna2, [λcI857 ΔBamΔH1], Δ[chlD-pgl], nadA::Tn10).

## EXAMPLE III

### Creation of Tryptophan Deficient Klebsiella aerogenes, GX1705

A host strain with a mutation in the trp operon was derived by chemical mutagenesis.

Klebsiella aerogenes strain LV025 (lsd) cells were grown in 20 ml LB broth at 30°C to $A_{540}$ = 0.45, centrifuged and resuspended in 20 ml of 0.1 M sodium citrate, pH 5.5. One ml of a freshly prepared and filter sterilized solution of nitrosoguanidine adjusted to a concentration of 1 mg/ml in citrate buffer pH 5.5, was added to the suspended cells. The cells were incubated at 30°C for 0.5 hours in the presence of nitrosoguanidine (indicated to kill approximately 50% of the K. aerogenes cells) and the cells were centrifuged at 4000 times g, washed with 0.1 M phosphate, (pH 7.0), resuspended in 50 ml LB and incubated with shaking overnight at 30°C.

Cycloserine treatment was then utilized to enrich for tryptophan auxotrophic mutants (see J. H. Miller, supra, pp. 230-234). One quarter of the above cells were isolated by centrifugation and resuspended in 100 ml minimal A medium containing 1% glucose and supplemented with all amino acids (20μg/ml) without glycine and without tryptophan. All minimal media cultures were supplemented with a standard solution of vitamins. The mixture was incubated at 30°C for 3-1/2 hours, 1 ml of freshly prepared 0.2 M D-cycloserine in 0.1 M phosphate buffer, pH 7.5 was added and the incubation continued for 3 hours in order to cause lysis of growing cells. Cells defective in glycine or tryptophan biosynthesis could not grow in this media and thus survived D-cycloserine treatment. The cells were isolated by centrifugation, resuspended in 50 ml of LB broth and incubated at 30° overnight. After a second cycloserine enrichment as described above, the cells were isolated and grown for a third treatment in 100ml minimal A 1% glucose media for 2 hours in the presence of 1 ml of 0.2 M D-cycloserine, after which the cells were again isolated by centrifugation and grown overnight in minimal A 1% glucose media with tryptophan and glycine at 20 µg/ml. A final cycloserine enrichment treatment was performed using the same procedure as for the third treatment. The cells were then plated on minimal A 1% glucose with 20 µg/ml tryptophan and replica plated on minimal A 1% glucose. Three mutants were identified that did not grow on minimal A 1% glucose in the absence of tryptophan. One of the three was also unable to grow on minimal A glucose with 10 µg/ml indole indicating that the mutation was in the region of the trp operon encoding tryptophan synthetase (trpBA). This line was labeled K.aerogenes GX1705 (lsd trp).

Formulations for the media as described above are as follows:

LB:  5 g/l yeast extract
     10 g/l tryptone
     5 g/l NaCl

Min A:    $K_2HPO_4$                    10.5 g/l
          $KH_2PO_4$                     4.5 g/l
          $(NH_4)_2SO_4$                 1.0 g/l
          Sodium citrate $2H_2O$         0.5 g/l
          $MgSO_4$                       0.2 g/l

100X Vitamin
Solution:     Biotin                        0.1  mg/ml
              Nicotinic acid                2.0  mg/ml
              Thiamine                      1.0  mg/ml
              Paraaminobenzoic acid         1.0  mg/ml
              Pantothenate                  5.0  mg/ml
              Pyridoxamine                  0.5  mg/ml


## Example IV

### Construction of a Compensating Plasmid for Expression of Serine Hydroxymethyltransferase, pGX2237 (trpEDCBA, glyA)


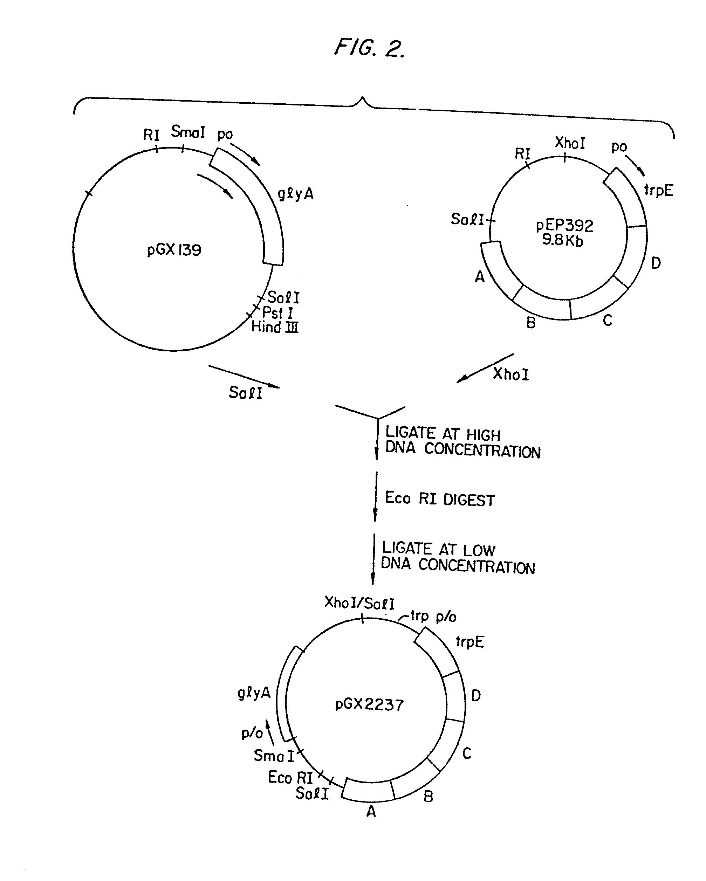

Plasmid pGX2237 was produced in the following manner (see Fig. 2). Plasmid pEP392 is described in detail in Emger-Valk, B.E. et al., Gene 9: 69-85 (1980).

Plasmid pEP392 (containing the nucleotide sequence coding for trpEDCBA regulated by the trp promoter, an Xho I restriction site upstream of the trp promoter, and an EcoRI restriction site upstream of the Xho I site) was linearized by treatment with Xho I. Plasmid pGX139 contains the E.coli glyA gene, including glyA regulatory region, which encodes the enzyme serine hydroxymethyltransferase (SHMT). Plasmid pGX139 was linearized by treatment with Sal I and ligated to the XhoI cut pEP392 using DNA ligase at high DNA concentration (1000 µg/ml). The resulting high molecular weight ligation polymer was digested with Eco RI to produce short linear DNA ligation products. The ligation products were circularized using DNA ligase at low DNA concentration (10 µg/ml).

Tryptophan deficient cells, K.aerogenes GX1705 from Example III were transformed with the ligation mixture and trp[+] transformant colonies, identified by replica plating on media with and without tryptophan, were chosen for further analysis. Plasmid DNA was isolated by known techniques and plasmids having the size predicted for pGX2237 were identified by gel electrophoresis. The corresponding transformant colonies were also analyzed for high SHMT production levels in enzyme assays. Samples of <u>Klebsiella</u> <u>aerogenes</u> GX1705 transformed with plasmid pGX2237 were deposited with the American Type Culture Collection in Rockville, Maryland, under accession number ATCC 39407 on 4 August 1983.

## Example V

### Evaluation of the Plasmid Stabilization Method With pGX2257, a Plasmid for Prochymosin Production with trpED Stabilization Genes

Plasmids pGX1049 and pGX2257 were transformed into E. coli strain GX1731 (see example II) using the calcium shock method (Mandel, M. and Higa, A., <u>Journal</u> <u>of</u> <u>Molecular</u> <u>Biology</u> <u>53</u>:159-162, 1970) selecting for ampicillin resistance. In a fermentation process it will not be feasible to use ampicillin as a selection mechanism. In order to determine the stabilization effect of the <u>trpED</u> genes on plasmid pGX2257, its stability was compared with plasmid pGX1049, a similar plasmid without tryptophan gene stabilization (see example I). GX1731 (pGX2257) was grown for >30 generations in casamino acid media (minimal A salts with 0.4% casamino acids) that lacks tryptophan using serial transfers in flasks. In media without tryptophan, there is positive selection for retention of pGX2257 by the <u>trp</u> mutant host cells. Likewise, GX1731 (pGX1049) was grown in casamino acid media plus tryptophan to supplement the <u>trpED</u> deletion in GX1731. In this case there is no positive selection for pGX1049 because ampicillin is not present. Plasmid stability was checked at various times by directly plating equal amounts of a GX1731(pGX2257) culture dilution on casamino acid media with tryptophan at 20 μg/ml (to determine total viable cells) and without tryptophan (to determine cells retaining plasmid). Likewise, a GX1731(pGX1049) culture dilution was plated on casamino acids media plus tryptophan at 20 µg/ml (to determine total viable cells) and casamino acid media plus tryptophan and ampicillin at 100 µg/ml (to determine cells retaining the plasmid). The results are shown in the table below.

|  |  | | %Stable |  |
| --- | --- | --- | --- | --- |
|  |  | | #colonies on selective media X100 |  |
|  |  | | #colonies on nonselective media |  |
|  |  | # Generations | Direct Plating Experiment | Replica Plating Experiment |
| GX1731 | (pGX1049) | 14 | 80 | 100 |
| GX1731 | (pGX1049) | 25 | 55 | 85 |
| GX1731 | (pGX1049) | 37 | 73 | 71 |
| GX1731 | (pGX2257) | 11 | 92 | 97 |
| GX1731 | (pGX2257) | 22 | 100 | 92 |
| GX1731 | (pGX2257) | 33 | 100 | 96 |

Because of the potential pipetting variation error inherent in a direct plating experiment, a second replica plating check for plasmid stability was also performed with the cultures. The data for this experiment is also shown in the Table. There is also potential error in the replica plating experiment since it is performed by picking 100 colonies plated on the nonselective media and then replicating them on selective media. The possibility exists for plasmid loss during the growth into a colony on the nonselective media. However, the data demonstrates that use of the trpED selection mechanism is a viable method for large scale fermentation where large volumes require many generations of growth.

## EXAMPLE VI

### Evaluation of the Plasmid Stabilization Method with Plasmid pGX2237, a Plasmid for Serine Hydroxymethyltransferase Production with trpEDCBA Gene Stabilization

A loop of Klebsiella aerogenes GX1705 cells transformed with pGX2237 (ATCC 39407 described in Examples III and IV) were inoculated into 100 ml of fermentation media and grown at 30°C in a 500 ml shake flask for 12 hours. Two identical seed cultures were inoculated into two 10 liter New Brunswick Scientific fermentors run at 30°C as described below. At the end of the run when growth had stopped, cells were diluted to various extents in sterile saline (0.15% NaCl) and plated in equal aliquots onto LB and minimal A glucose agar plates (Miller, Experiments in Molecular Genetics, Cold Spring Harbor Laboratory, 1972 pp. 432-433; also see example III).

Plating Experiment

| Fermentation | Test Dilution | # Colonies LB | Minimal A Glucose |
|---|---|---|---|
| MF1-6 | 1 | 32 | 32 |
| | 2 | 182 | 270 |
| MF1-8 | 1 | 56 | 43 |
| | 2 | 299 | 375 |

The number of colonies on minimal plates was comparable to the number on LB plates suggesting no plasmid loss problem. In order to further examine this question, a replica plating experiment was performed. One hundred colonies from the LB plates above were picked with sterile toothpicks and placed onto a second LB plate and on to a minimal A glucose plate and grown at 30°C.

Replica Plating Experiment

| Fermentation | LB | # Colonies Minimal A Glucose |
|---|---|---|
| MF1-6 | 100 | 100 |
| MF1-8 | 100 | 99 |

Only one colony of 200 failed to grow on the minimal plate. As these colonies were first grown on the LB plate of the plating experiment (non-selective condition with tryptophan present) failure of one colony to grow is not unexpected. Thus, plasmid stability was maintained during 10-liter scaled-up fermentation runs.

Fermentation media:

$K_2 HPO_4$    5.25 g/l
$KH_2 PO_4$    2.25 g/l
sodium citrate 0.5 g/l
100 X trace minerals 10 ml/1
$(NH_4)_2 SO_4$    10 ml/1
$FeSO_4 \cdot 7H_2O$   $1 \times 10^{-4}$ g/l
$MgSO_4 \cdot 7H_2O$   $5.7 \times 10^{-2}$ g/l
sucrose 20 g/l

100 X Trace Minerals

$CaCL_2 \cdot 2H_2O$   1.1 g/l
$FeSO_4 \cdot 7H_2O$   0.7 g/l
$MnSO_4 \cdot 4H_2O$   0.2 g/l
$ZnSO_4 \cdot 7H_2O$   0.2 g/l
$CuSO_4 \cdot 5H_2O$   0.04 g/l
disodium
ethylenediaminetetraacetate   39.4 g/l

Fermentation Conditions

Using a New Brunswick scientific microferm fermenter (MF-114), the pH was maintained at 7.0 by automatic addition of 10% $NH_4OH$ and 10% $H_2SO_4$. Agitation was at 600 rpm, temperature was 30°C, and aeration was 1 volume air per volume media per minute.

Claims

1. A method for the large scale production of a protein by a microorganism which comprises transforming an auxotrophic strain of said microorganism, having at least one mutation in the tryptophan operon such that the cells are unable to produce sufficient tryptophan to survive or grow, with a recombinant vector which comprises (a) a gene which encodes a protein of interest and is under the control of regulatory sequences for expression and (b) one or more functional trp genes, under the control of regulatory sequences for expression, which at least complement the mutation(s) in the tryptophan operon of the host cells thereby enabling the said host cells to synthesize tryptophan, and growing the transformed cells under gene expressing conditions in nutrient medium therefor substantially free of tryptophan.

2. A method of stabilizing a host microorganism / expression vector system for large scale production of a protein which comprises transforming an auxotrophic strain of said microorganism, having at least one mutation in the tryptophan operon such that the cells are unable to produce sufficient tryptophan to survive or grow, with a recombinant vector which comprises (a) a gene which encodes a protein of interest and is under the control of regulatory sequences for expression and (b) one or more functional trp genes, under the control of regulatory sequences for expression, which at least complement the mutation(s) in the tryptophan operon of the host cells thereby enabling the said host cells to synthesize tryptophan.

3. A method as claimed in claim 1 or claim 2 which includes the step of creating at least one mutation in the tryptophan operon of said microorganism.

4. A method as claimed in any one of claims 1 or 3 wherein the auxotrophic host cells have a deletion in the tryptophan operon.

5.     A method as claimed in any one of claims 1 to 3 wherein the auxotrophic host cells have a mutation in the tryptophan operon which causes improper translation.

6.     A method as claimed in any one of claims 1 to 3 wherein the auxotrophic host cells have a mutation in the tryptophan operon which interrupts either transcription or translation.

7.     A method as claimed in claim 3 wherein mutation is effected by use of ultraviolet light or a chemical mutagen.

8.     A method as claimed in claim 3 wherein mutation is induced by a virus or by a transposon.

9.     A method as claimed in claim 4 wherein the deleted portion of the tryptophan operon is the trpED region.

10.     A method as claimed in any one of claims 1 to 9 wherein the nutrient medium is one in which tryptophan has been degraded.

11.     A host microorganism / expression vector system stablized by a method as claimed in claim 2.

12.     The stabilized host microorganism / expression vector system of claim 11 wherein the protein of interest encoded by the expression vector is prochymosin.

13.     The stablized host microorganism / expression vector system of claim 11 wherein the protein of interest encoded by the expression vector is serine hydroxymethyltransferase.

14.     E. coli strain GX1731, transformed with pGX2257 and designated NRRL B-15771.

15.     Klebsiella aerogenes strain GX1705 transformed with PGX2237 and designated ATCC 39407.

16.     A recombinant vector for use in a method as claimed in claim 1 or claim 2 which comprises (a) a gene which encodes a protein of interest and is under the control of regulatory sequences for expression and (b) a functional trp gene or genes under the control of regulatory sequences for expression.

Figure 1

FIG. 2.

0178764

**European Patent Office**

**EUROPEAN SEARCH REPORT**

Application number

| DOCUMENTS CONSIDERED TO BE RELEVANT | | EP 85306061.4 |

| Category | Citation of document with indication, where appropriate, of relevant passages | Relevant to claim | CLASSIFICATION OF THE APPLICATION (Int. Cl.4) |
|---|---|---|---|
| P,A | EP - A1 - 0 134 662 (GENEX CORPORATION)<br>* Claims 1,4,14 * | 1,12, 13,16 | C 12 N 15/00<br>C 12 P 21/00<br>C 12 N 1/20<br>//C 12 R 1:19<br>C 12 R 1:22 |
| D,A | US - A - 4 371 615 (MIWA et al.)<br>* Claims 1,8 * | 1,2 | |
| A | EP - A2 - 0 036 776 (GENENTECH, INC.)<br>* Abstract * | 1,16 | |
| A | JOURNAL OF BACTERIOLOGY, vol. 133, March 1978 (Washington DC)<br>G.F. MIOZZARI et al. "Translation of the Leader Region of Escherichia coli Tryptophan Operon" pages 1457-1466<br>* Totality * | 1 | |

| | | | TECHNICAL FIELDS SEARCHED (Int. Cl.4) |
|---|---|---|---|
| | | | C 12 N<br>C 12 P |

The present search report has been drawn up for all claims

| Place of search | Date of completion of the search | Examiner |
|---|---|---|
| VIENNA | 22-01-1986 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82